# EUROPÄISCHE PATENTANMELDUNG

(11) **EP 2 407 104 A2**
(43) Veröffentlichungstag der Anmeldung: **18.01.2012**
(21) Anmeldenummer: 11172369.8
(22) Anmeldetag: 01.07.2011
(51) Int. Cl.: A61B 5/145

(54) **Implantierbare Sensoreinrichtung**

(30) Priorität: 16.07.2010 US 364814 P
(71) Anmelder: Dyconex AG, 8303 Bassersdorf (CH)
(72) Erfinder: Dörr, Thomas, 12437 Berlin (DE); Hauer, Marc, 8050 Zürich (CH)
(74) Vertreter: Lindner-Vogt, Karin L.

(57) **Zusammenfassung**

Implantierbare Sensoreinrichtung (100) zur Erfassung mindestens eines physikalischen, chemischen, biologischen oder physiologischen Parameters im Körper eines die Sensoreinrichtung tragenden Lebewesens im Kontakt mit dessen Körperflüssigkeit oder -gewebe mit einem Sensorgehäuse (110), einem in dem Sensorgehäuse untergebrachten Sensorelement (120), das einen Erfassungsabschnitt aufweist, der direkt im Kontakt mit der Körperflüssigkeit oder dem Körpergewebe steht oder zu einem im Kontakt mit der Körperflüssigkeit oder dem Körpergewebe stehenden Oberflächen- oder Öffnungsabschnitt des Sensorgehäuses innenseitig benachbart ist, und einer mechanisch wirkenden Sensor-Reinigungseinrichtung (425) zur Reinigung des Erfassungsabschnitts des Sensorelements bzw. des zu diesem benachbarten Oberflächenabschnitts des Sensorgehäuses.

## Beschreibung

Die Erfindung betrifft eine implantierbare Sensoreinrichtung zur Erfassung mindestens eines physikalischen, chemischen, biologischen oder physiologischen Parameters im Körper eines die Sensoreinrichtung tragenden Lebewesens im Kontakt mit dessen Körperflüssigkeit oder -gewebe.

Derartige Sensoreinrichtungen sind, vor allem als Signalgeber für gleichfalls implantierte medizinelektronische Geräte, aber auch als Signalgeber zur externen Patientenüberwachung, seit langem in vielgestaltigen Ausführungen bekannt und auch im klinischen Einsatz. Besonders verbreitet sind Abfühlelektroden zum Abgriff elektrischer Potentiale im Körper, etwa von Herz- oder anderen Muskelaktionspotentialen oder Hirnströmen. Bekannt und zumindest punktuell im Einsatz befindlich sind aber auch nicht-elektrische Sensoren, wie etwa optische Sensoren zur Erfassung der Blutsauerstoffsättigung, Druckfühler zur Erfassung des Blut- bzw. Gefäßinnendrucks, elektrochemische Sensoren und andere.

Vor allem bei den nicht-elektrischen Sensortypen hat sich im Dauereinsatz vielfach das Problem gezeigt, dass die Sensoroberfläche mit körpereigenem Gewebe zuwächst bzw. sich aus umgebender Körperflüssigkeit denaturierte Proteine auf der Sensoroberfläche absetzen. Hierdurch wird zumindest die Empfindlichkeit des Sensors in nachteiliger Weise reduziert, häufig geht aber sogar die Funktionsfähigkeit völlig verloren und der Sensor wird unbrauchbar.

Als Gegenmaßnahme hiergegen sind verschiedene Beschichtungen der Sensoroberflächen entwickelt worden, die derart ausgebildet sind, dass die biochemische Anlagerungskette gestört wird. Darüber hinaus wurde der Ansatz eines "Freibrennens" der Erfassungsoberflächen von speziell im Hinblick auf die Reinigungsfunktion gestalteten Sensorarrays verfolgt. Diese Ansätze haben sich als nur bedingt erfolgreich erwiesen.

Der Erfindung liegt daher die Aufgabe zugrunde, eine verbesserte Sensoreinrichtung der genannten Art anzugeben, die im Dauereinsatz eine verbesserte Zuverlässigkeit und längere Lebensdauer aufweist.

Diese Aufgabe wird mit einer Sensoreinrichtung mit den Merkmalen des Anspruchs 1 gelöst. Zweckmäßige Fortbildungen es Erfindungsgedankens sind Gegenstand der abhängigen Ansprüche.

Mit der Erfindung wird vorgeschlagen, in der Sensoreinrichtung eine mechanisch wirkenden Sensor-Reinigungseinrichtung zur Reinigung des Erfassungsabschnitts des Sensorelements bzw. des zu diesem benachbarten Oberflächenabschnitts des Sensorgehäuses vorzusehen.

In einer Ausführung der Erfindung weist die Sensor-Reinigungseinrichtung Gasblasen-Erzeugungsmittel auf, welche derart ausgebildet und angeordnet sind, dass Gasblasen im Erfassungsabschnitt des Sensorelements oder dem hierzu benachbarten Oberflächenabschnitt des Sensorgehäuses erzeugt oder über diesen bewegt werden.

Diese Ausführung ist in verschiedenartiger Weise weiter auszugestalten. In einer Ausgestaltung ist vorgesehen, dass elektrothermisch wirkende Gasblasen-Erzeugungsmittel vorgesehen sind, die insbesondere eine Reinigungsstrom-Erzeugungseinrichtung und eine mit dieser verbundene Leiteranordnung oder Leitschicht aufweisen. Eine weitere Ausgestaltung sieht vor, dass die Gasblasen-Erzeugungsmittel einen Ultraschallerzeuger, insbesondere einen mit einem Ultraschallgenerator verbundenen Piezoschwinger, aufweisen.

In einer weiteren Ausführung sind elektrochemisch wirkende Gasblasen-Erzeugungsmittel vorgesehen, die insbesondere mindestens eine Elektrode und Gegenelektrode und eine mit diesen verbundene Reinigungsstrom-Erzeugniseinrichtung aufweisen. Ähnlich konstruiert ist eine Ausgestaltung der Gasblasen-Erzeugungsmittel, wobei die Gasblasen-Erzeugungsmittel zum Bewirken einen Hochspannungsüberschlags ausgebildet sind und die insbesondere mindestens eine Elektrode und Gegenelektrode und eine mit diesen verbundener Reinigungsspannungs-Erzeugungseinrichtung aufweist.

Sowohl die elektrochemisch wirkende als auch die Hochspannungs-Gasblasen-Erzeugungseinrichtung können zweckmäßig in einer weiteren Ausgestaltung eingesetzt werden. Bei dieser sind der Erfassungsabschnitt und mindesten eine der Elektroden in einem Lumen des Sensorgehäuses mit einer Öffnung angeordnet derart, dass die durch eine elektrochemische Reaktion oder den Hochspannungsüberschlag bewirkte Gasbildung eine Druckwelle im Lumen erzeugt, die sich durch die Öffnung ausbreitet.

Im Hinblick auf die Vermeidung physiologischer Probleme beim Betrieb der Sensor-Reinigungseinrichtung sind in einer weiteren Ausführung die Gasblasen-Erzeugungsmittel derart ausgebildet, dass sie Gasblasen mit einem mittleren Volumen von weniger als 1 ml erzeugen.

Eine weitere Ausführung sieht vor, dass die Sensor-Reinigungseinrichtung Zeitsteuermittel zu zeitabhängigen, insbesondere periodischen, Aktivierung der Reinigungsfunktion aufweisen. Hierdurch kann der (typischerweise die Batterie der Sensoreinrichtung belastende) Energieverbrauch der Reinigungsvorgänge unter Heranziehung der Erfahrungswerten bezüglich der Zeitdauer des Aufwachsens von Blut- bzw. Gewebsbestandteilen minimiert werden. In einer hierzu alternativen oder auch mit der vorgenannten Ausführung kombinierbaren Variante weist die Sensor-Reinigungseinrichtung sensorsignal-abhängige Steuermittel zu Aktivierung der Reinigungsfunktion im Ansprechen auf eine anormale Zeitabhängigkeit der Signale des Sensorelements auf. Diese letztere Variante erfordert zwar einen höheren Implementierungsaufwand, erlaubt aber eine noch bessere Anpassung der Reinigungsfunktion an die tatsächliche Beaufschlagung der Sensoroberfläche im Betrieb der Sensoreinrichtung.

Vorteile und Zweckmäßigkeiten der Erfindung ergeben sich im Übrigen aus der nachfolgenden Beschreibung von Ausführungsbeispielen anhand der Figuren. Von diesen zeigen:
- Fig. 1A und 1B: schematische Darstellungen eines passiven Sensorimplantats,
- Fig. 2: eine synoptische Darstellung zur Verdeutlichung des durch die Erfindung zu lösenden Problems,
- Fig. 3: eine schematische Darstellung einer Ausführungsform der Erfindung,
- Fig.4: eine schematische Darstellung einer weiteren Ausführungsform der Erfindung,
- Fig. 5: eine schematische Darstellung einer weiteren Ausführungsform der Erfindung,
- Fig. 6: eine schematische Darstellung einer weiteren Ausführungsform der Erfindung,
- Fig. 7: eine schematische Darstellung einer weiteren Ausführungsform der Erfindung,

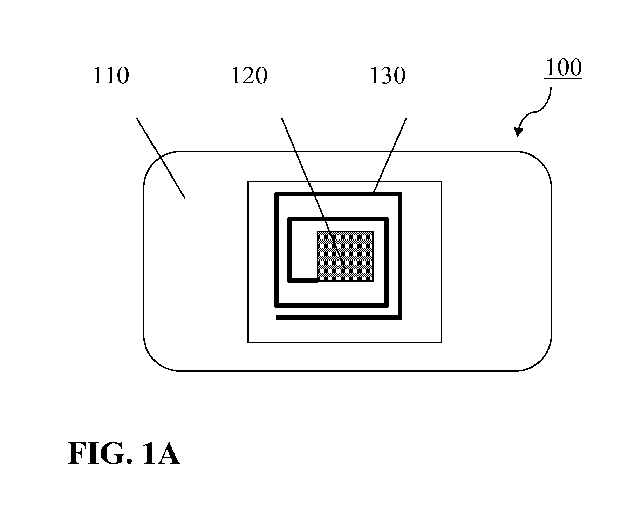

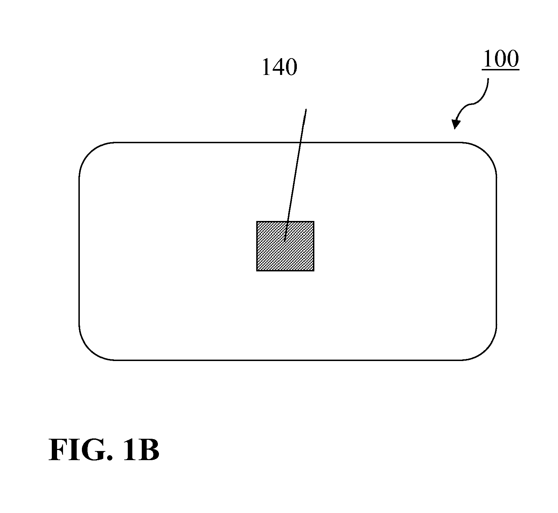

Fig. 1A und 1B zeigen in Art von Draufsichten schematisch den Grundaufbau eines passiven Sensorimplantats bzw. einer implantierbaren Sensoreinrichtung 100 mit einem Sensorgehäuse 110, einem Sensorelement 120 und einer Spule 130 zur Signalübertragung im Zusammenhang mit einer telemetrischen Aktivierung des Sensors und Kommunikation mit einem außerhalb des Körpers eines Trägers befindlichen Telemetriegerät. Fig. 1B zeigt, dass der Sensor im Gebrauchszustand vom Sensorgehäuse 110 hermetisch umschlossen ist, wobei nur ein für das Gewinnen der Sensorinformation im Kontakt mit Körperflüssigkeit oder Körpergewebe des Trägers benötigtes Sensorfenster bzw. ein Erfassungsabschnitt 140 nicht eingekapselt ist.

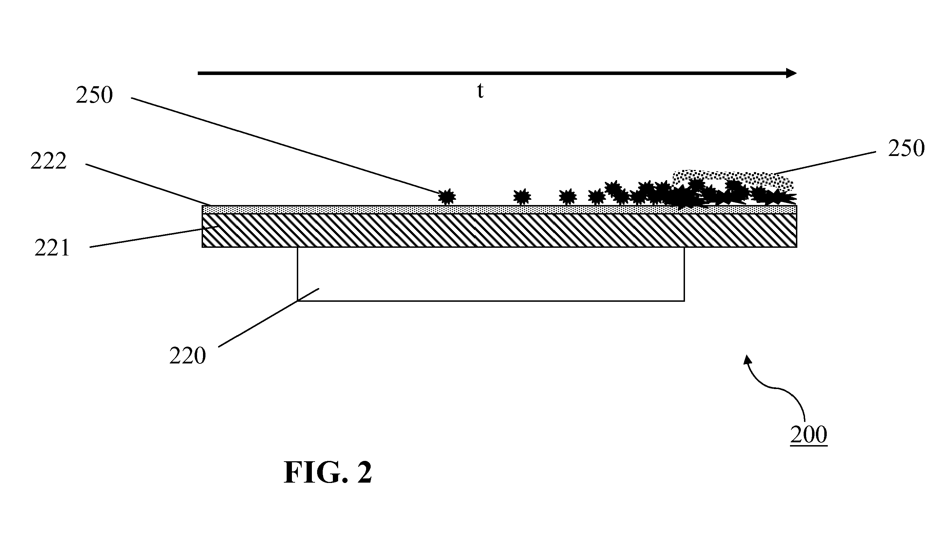

Mit Fig. 2 wird das Problem verdeutlicht, das bei derartigen implantierbaren Sensoreinrichtungen im Langzeiteinsatz im Körper eines Trägers auftritt und das Anlass für die Überlegungen der Erfinder gegeben hat. Ein hier als "Blackbox" gezeigtes und mit Ziffer 220 bezeichnetes Sensorelement ist mit einer für die Sensorinformation transparenten Schutzschicht 221 abgedeckt, welche wiederum eine spezielle Oberflächenschicht 222 aufweist, die ein Aufwachsen von biologischem Material hemmt. Im Verlaufe der Zeit - hier mit einer Zeitachse t symbolisiert - degenerieren aber auch an dieser speziellen Oberfläche 222 körpereigener Proteine und führen zu Auflagerungen 250, die das Sensorelement 220 zunehmend gegenüber den die Sensorinformationen beinhaltenden Körperflüssigkeiten abkapseln und damit unbrauchbar machen.

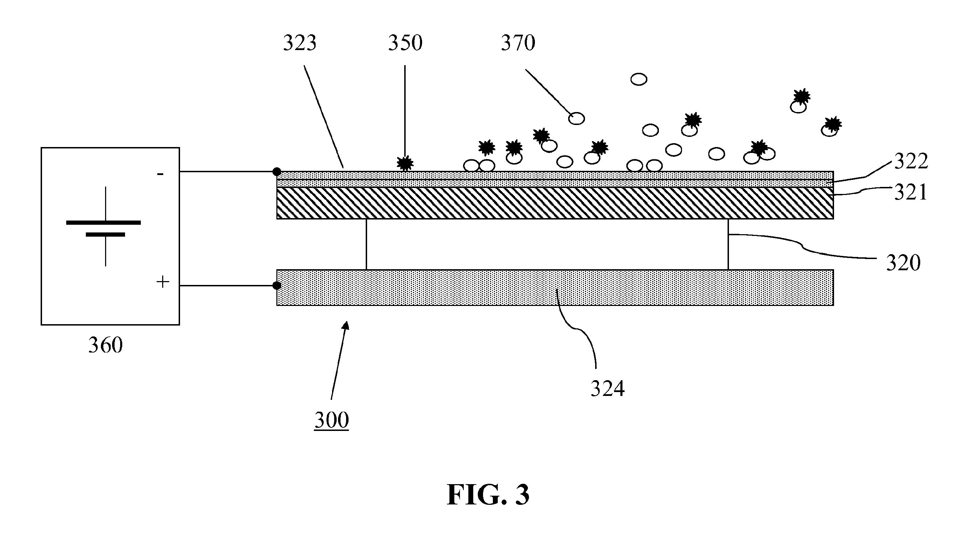

Fig. 3 zeigt in einer ähnlichen Art der Darstellung wie Fig. 2 den Grundaufbau einer ersten erfindungsgemäßen Sensoreinrichtung 300 mit einem Sensorelement 320, welcher über der transparenten Schutzschicht 321 und der bewuchshemmenden Oberflächenschicht 322 zusätzlich eine elektrisch leitfähige Oberfläche 323 besitzt, die für die Sensorinformation ebenfalls transparent ist. Mittels einer auf der Rückseite des Sensorelements 320 vorgesehenen Gegenelektrode 324 und einer steuerbaren Stromquelle 360 wird an die Oberfläche (den Erfassungsabschnitt) der Sensoreinrichtung in periodischen Abständen eine elektrische Spannung angelegt. Diese Spannung wird, im Abstimmung auf die elektrischen Parameter der Elektrodenschichten 322, 324 und die die Sensoreinrichtung typischerweise umgebenden Körperflüssigkeiten so gewählt, dass ein zum Bewirken einer elektrochemischen Reaktion und der Entwicklung von Gasblasen 370 an die Erfassungsfläche hinreichender Strom fließt.

Die Gasblasen 370 lösen während der periodischen Reinigungsvorgänge zwischenzeitlich angelagerte degenerierte Proteine 350 von der Erfassungsfläche ab. Die Dauer und Intensität der Stromeinprägung werden dabei so fest gelegt, dass eine kurzzeitige Blasenbildung über die gesamte Erfassungsfläche erfolgt, jedoch das umliegende Körpergewebe nicht geschädigt wird und die erzeugte Gasmenge physiologischen bedenklich ist.

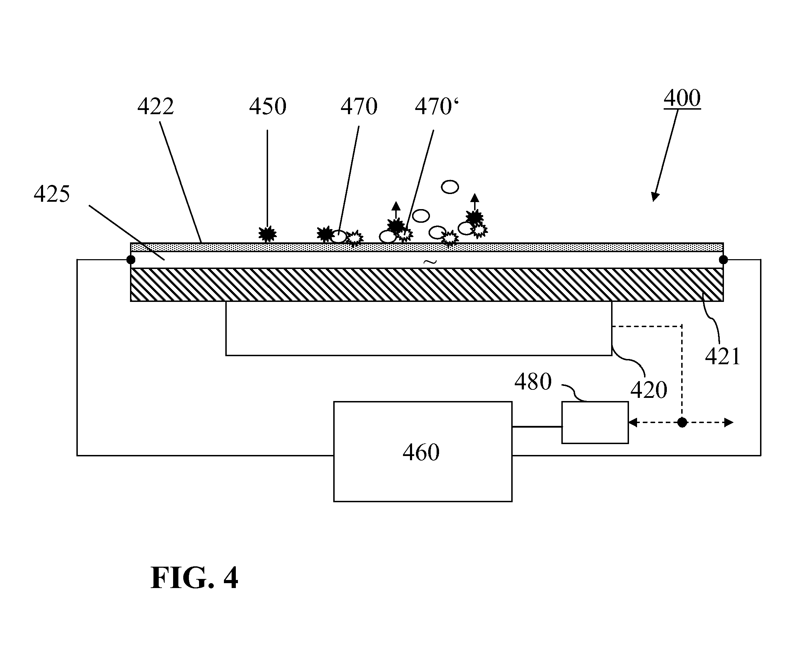

Fig. 4 zeigt als weitere Ausführung der Erfindung eine Sensoreinrichtung 400, welche wiederum ein Sensorelement 420 und die übliche transparente Schutzschicht 421 umfasst, bei bei der jedoch anders funktionierende Gasblasen-Erzeugungsmittel als bei der ersten Ausfiihrungsform vorgesehen sind.

Diese Mittel umfassen hier einen Piezoschwinger 425, der von einem Ultraschallgenerator 460 periodisch jeweils zu einem Reinigungszyklus im Ultraschallbereich zum Schwingen angeregt wird. Der Piezoschwinger 425 ist auf der Oberfläche der herkömmlichen transparenten Schutzschicht 421 angeordnet, und die bewuchshemmende Oberflächenbeschichtung 422 ist hier auf dem Piezoschwinger 425 angeordnet. Die Ultraschallschwingungen generieren auf der Sensoroberfläche kleine Gasblasen 470, die unmittelbar nach ihrer Entstehung kollabieren (vgl. Ziffer 470') und dabei zwischenzeitlich angelagerte Proteine 450 von der Erfassungsfläche ablösen. Das Wirkprinzip dieser Ausführung der Erfindung entspricht mindestens teilweise demjenigen eines Ultraschall-Reinigungsbades.

Als Steuereinrichtung zur Ansteuerung der Reinigungsprozeduren ist im Ultraschallgenerator eine Steuereinrichtung 480 zugeordnet, die im einfachsten Fall als Timer ausgeführt, in einer intelligenteren (mit gestrichelten Linien dargestellten) Ausgestaltung aber eingangsseitig mit dem Sensorelement 420 verbunden sein und eine Degeneration des Sensorsignals erfassen und zum Auslöser zum Starten eines Reinigungsvorganges nehmen kann.

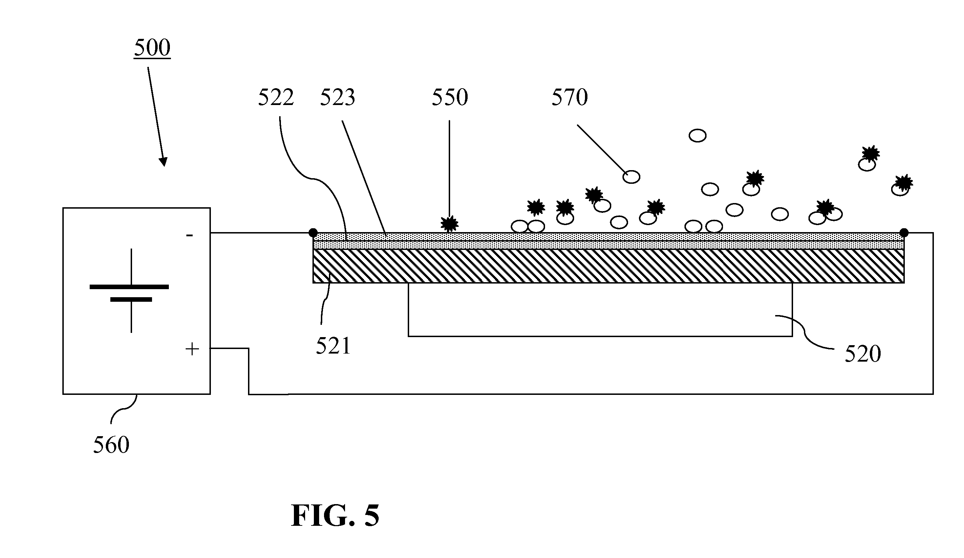

Fig. 5 zeigt eine der Ausführung nach Fig. 2 sehr ähnliche Variante der Erfindung, bei der konstruktiv im Grunde nur die Gegenelektrode entfällt. Die übrigen Teile entsprechen der Ausführung nach Fig. 3 und sind mit hieran angelehnten Bezugsziffern bezeichnet. Bei der Oberflächenschicht 523 handelt es sich um eine für das Sensorsignal transparente Leitschicht, die sich bei Durchgang eines kurzzeitig angelegten, relativ hohen Stroms so stark erwärmt, dass sich in der umgebenden Körperflüssigkeit Gasblasen 570 bilden.

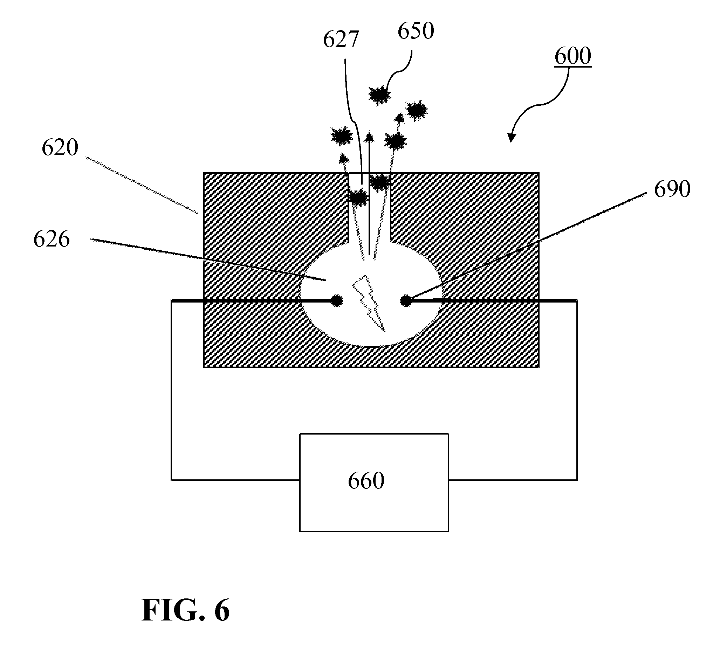

Ein andersartiges Wirkprinzip hat die weitere Sensoreinrichtung 600 nach Fig. 6. Deren Sensorelement 620 hat ein Lumen 626, in dem sich ein (hier nicht gesondert bezeichneter) Erfassungsabschnitt des Sensorelements befindet, mit einer Öffnung 627, durch die das Lumen mit den umgebenden Körperflüssigkeiten des Implantat-Trägers in Flüssigkeitsverbindung steht. Um diese Öffnung regelmäßig zu reinigen, sind im Lumen 626 zwei Elektroden 690 angeordnet, die mit einem Hochspannungsgenerator 660 verbunden sind und von diesem zu Reinigungszyklen periodisch mit Hochspannung versorgt werden. Die dabei entstehende explosionsartige Gasbildung erzeugt im Lumen 626 einen erheblichen Überdruck, durch den die Öffnung 627 verstopfende denaturierte Proteine 650 herausgeschleudert werden. Hierdurch wird die Öffnung des Sensorelements 620 zuverlässig gereinigt.

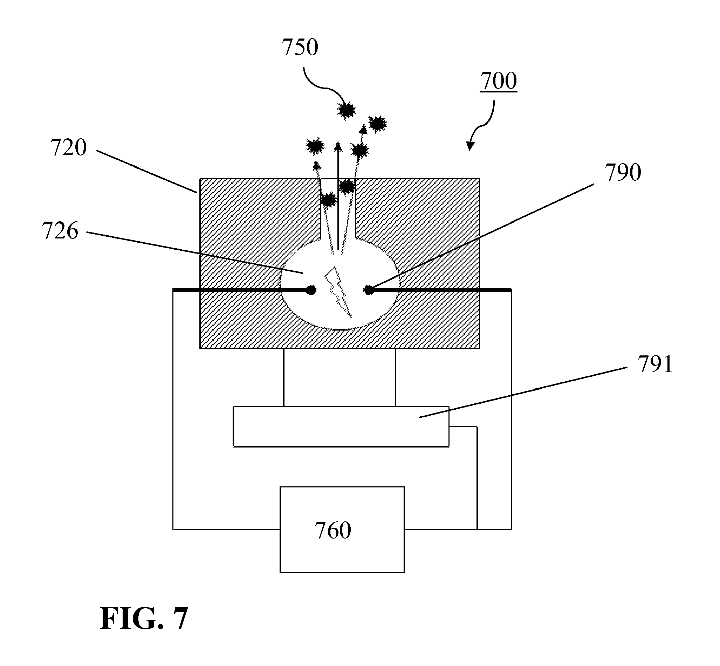

Fig. 7 zeigt als abgewandelte Ausführung der zuletzt beschriebenen Ausführung eine Sensoreinrichtung 700 mit im Wesentlichen gleichem Aufbau wie die Sensoreinrichtung 600 nach Fig. 6, bei der aber ein anderer Reinigungsmechanismus eingesetzt wird. Die Erzeugung der Gase im Lumen 726 erfolgt hier durch eine elektrochemische Gasbildung an einer der beiden Elektroden 790. Die andere der beiden Elektroden kann die erforderliche Gegenelektrode bilden, es kann aber auch - wie in der Fig. in synoptischer Weise zusätzlich gezeigt - eine außerhalb des Lumens 726 befindliche Gegenelektrode 791 vorgesehen sein.

Die Ausführung der Erfindung ist nicht auf die oben hervorgehobenen Aspekte und beschriebenen Beispiele beschränkt, sondern kann ebenso in einer Vielzahl von Abwandlungen erfolgen, die im Rahmen fachgemäßen Handelns liegen.

## Patentansprüche

1. Implantierbare Sensoreinrichtung zur Erfassung mindestens eines physikalischen, chemischen, biologischen oder physiologischen Parameters im Körper eines die Sensoreinrichtung tragenden Lebewesens im Kontakt mit dessen Körperflüssigkeit oder Körpergewebe, mit
einem Sensorgehäuse,
einem in dem Sensorgehäuse untergebrachten Sensorelement, das einen Erfassungsabschnitt aufweist, der direkt im Kontakt mit der Körperflüssigkeit oder dem Körpergewebe steht oder zu einem im Kontakt mit der Körperflüssigkeit oder dem Körpergewebe stehenden Oberflächen- oder Öffnungsabschnitt des Sensorgehäuses innenseitig benachbart ist, und
einer mechanisch wirkenden Sensor-Reinigungseinrichtung zur Reinigung des Erfassungsabschnitts des Sensorelements bzw. des zu diesem benachbarten Oberflächenabschnitts des Sensorgehäuses.

2. Sensoreinrichtung nach Anspruch 1, wobei die Sensor-Reinigungseinrichtung Gasblasen-Erzeugungsmittel aufweist, welche derart ausgebildet und angeordnet sind, dass Glasblasen im Erfassungsabschnitt des Sensorelements oder dem hierzu benachbarten Oberflächenabschnitt des Sensorgehäuses erzeugt oder über diesen bewegt werden.

3. Sensoreinrichtung nach Anspruch 2, wobei elektro-thermisch wirkende Gasblasen-Erzeugungsmittel vorgesehen sind, die insbesondere eine Reinigungsstrom-Erzeugungseinrichtung und eine mit dieser verbundene Leiteranordnung oder Leitschicht aufweisen.

4. Sensoreinrichtung nach Anspruch 2, wobei die Gasblasen-Erzeugungsmittel einen Ultraschallerzeuger, insbesondere einen mit einem Ultraschallgenerator verbundenen Piezoschwinger, aufweisen.

5. Sensoreinrichtung nach Anspruch 2, wobei elektrochemisch wirkende Gasblasen-Erzeugungsmittel vorgesehen sind, die insbesondere mindestens eine Elektrode und Gegenelektrode und eine mit diesen verbundene Reinigungsstrom-Erzeugniseinrichtung aufweisen.

6. Sensoreinrichtung nach Anspruch 2, wobei die Gasblasen-Erzeugungsmittel zum Bewirken eines Hochspannungsüberschlags ausgebildet sind und insbesondere mindestens eine Elektrode und Gegenelektrode und eine mit diesen verbundener Reinigungsspannungs-Erzeugungseinrichtung aufweisen.

7. Sensoreinrichtung nach Anspruch 5 oder 6, wobei der Erfassungsabschnitt und mindesten eine der Elektroden in einem Lumen des Sensorgehäuses mit einer Öffnung angeordnet sind derart, dass die durch eine elektrochemische Reaktion oder den Hochspannungsüberschlag bewirkte Gasbildung eine Druckwelle im Lumen erzeugt, die sich durch die Öffnung ausbreitet.

8. Sensoreinrichtung nach Anspruch 2, wobei die Gasblasen-Erzeugungsmittel derart ausgebildet sind, dass sie Gasblasen mit einem mittleren Volumen von weniger als 1 ml, erzeugen.

9. Sensoreinrichtung nach einen der vorangehenden Ansprüche, wobei die Sensor-Reinigungseinrichtung Zeitsteuermittel zu zeitabhängigen, insbesondere periodischen, Aktivierung der Reinigungsfunktion aufweisen.

10. Sensoreinrichtung nach einen der vorangehenden Ansprüche, wobei die Sensor-Reinigungseinrichtung sensorsignal-abhängige Steuermittel zu Aktivierung der Reinigungsfunktion im Ansprechen auf eine anormale Zeitabhängigkeit der Signale des Sensorelements aufweisen.
